# EUROPEAN PATENT SPECIFICATION

(11) **EP 2 875 845 B1**
(45) Date of publication and mention of the grant of the patent: **24.02.2016**
(21) Application number: 14189466.7
(22) Date of filing: 20.10.2014
(51) Int. Cl.: A61N 1/372, A61N 1/375

(54) **Sterilizable containment for implantable medical device**
Sterilisierbarer Behälter für implantierbare medizinische Vorrichtung
Confinement stérilisable pour dispositif médical implantable

(30) Priority: 21.11.2013 US 201361906902 P; 14.05.2014 US 201461992930 P
(43) Date of publication of application: 27.05.2015
(73) Proprietor: BIOTRONIK SE & Co. KG, 12359 Berlin (DE)
(72) Inventor: Taff, Brian M., Portland, OR Oregon 97217 (US); Kraetschmer, Hannes, West Linn, OR Oregon 97068 (US); von Arx, Jeffrey A., Lake Oswego, OR Oregon 97035 (US)
(74) Representative: Galander, Marcus

(56) References cited:
- FR-A3- 2 483 215
- US-A- 4 423 732
- US-A- 4 588 085
- US-A- 6 116 413
- US-A1- 2002 161 402
- US-A1- 2006 020 300
- US-A1- 2007 123 947
- US-A1- 2009 124 965
- US-A1- 2010 004 639
- US-A1- 2010 114 247
- US-B1- 6 181 105
- US-B1- 6 292 697

## Description

The invention refers to a sterilizable containment for an implantable medical device. The invention further refers to a sterilizable containment containing an implantable medical device. In particular, the invention refers to a sterilizable containment for an implantable programmable heart stimulator, such as an implantable pacemaker or an implantable cardioverter/defibrillator. The invention further refers to a method of communicating with an implantable medical device while placed in the containment.

Implantable medical devices must be sterilized prior to use. Therefore, a containment is needed, wherein the implantable medical device can be maintained sterilized. To ensure that the implantable medical device remains sterile, the implantable medical device is sterilized together with a containment enclosing the device.

Examples of sterilizable containments, that comprise an inner packaging and an outer packaging, are disclosed in documents US 4,423,732 A, US 2007/0123947 A1, US 6,181,105 B1, US 4,588,085 A or FR 2 483 215 A3. In these documents the outer packaging encloses the inner packaging and comprises at least two electric feedthroughs. The inner packaging comprises at least two electric contacts, each of the contacts of the inner packaging matches one of the feedthroughs of the outer packaging to provide an electric connection between a respective feedthrough and the corresponding contact when the inner and the outer packaging are closed. Each of the contacts of the inner packaging is configured and arranged to provide electrical conductivity or capacitive coupling from outside of the inner packaging to the inside of the inner packaging.

Another example of a sterilizable container is disclosed in document US 6,116,413 A, which discloses the use of a container with conductive liquid containing a medical device. One example for implantable medical devices are implantable leadless pacemakers (ILP) that are small enough to be placed within a ventricle of a human heart. Such implantable leadless pacemakers typically feature two electrodes that are arranged on the pacemaker's casing and that are configured to deliver stimulation pulses to a medium or tissue surrounding the implantable leadless pacemaker when in use. Traditional pacemakers are implanted distal to the heart and connected to separately implanted electrode leads with electrodes for sensing and stimulation within the heart. For implantable leadless pacemakers, no electrode leads are needed and therefore only one device needs to be implanted and kept sterile prior to implantation.

Implantable medical devices can comprise a communication unit for communication to an external device like a programmer for programming of the device and interrogation of device data. Traditional pacemakers often use wireless communication with radio frequencies or by magnetic coupling by coils. Such communication techniques require a significant amount of energy for transmission and size for antennas or communication coils that may be not available in small implantable medical devices like implantable leadless pacemaker (ILP). Alternative communication techniques more suitable for example for implantable leadless pacemaker (ILP) are based on a galvanic or electrical conductive coupling between the implantable medical device and the external device. In general, galvanic communication is referred to a communication that is based on alterations of electric charges, either by altering capacitive coupled small electric fields or by altering a current of voltage via a galvanic connection between transmitter and receiver. This requires an electrical conductivity or capacitive coupling between the programmer and the implantable medical device. In implanted state the body provides such an electrical conductivity or capacitive coupling between electrodes of the implanted medical device and tissue surface electrodes connected to the external device.

A further alternative communication technique more suitable for example for implantable leadless pacemaker (ILP) is based on a small electric field imposed on the implantable medical device. Data are transmitted to the implantable medical device by modulating the electric field. The implantable medical device on the other hand comprises means to transmit data by altering the imposed electric field, e.g. by generating an electric field between the device electrodes. This requires only a capacitive coupling of electrodes connected to the external device to the implantable medical device.

It is desired to enable an electrical conductive or capacitive coupling to the implantable medical device for programming prior to the implantation when the device is in a sterile containment.

It is an object of the invention to provide a suitable containment for an implantable medical device, and in particular for a programmable implantable leadless pacemaker.

According to the invention, this object is achieved by means of a sterilizable containment that comprises an inner packaging and an outer packaging. The outer packaging encloses the inner packaging and comprises at least two electric feedthroughs. The inner packaging comprises at least two electric contacts, each of the contacts of the inner packaging matches one of the feedthroughs of the outer packaging to provide an electric connection between a respective feedthrough and the corresponding contact when the inner and the outer packaging are closed. It is to be understood that the containment and the inner and the outer packaging are opened prior to insertion of an implantable medical device into the containment. The implantable medical device will be placed in the inner packaging, and then the inner and the outer packaging are closed. Afterwards, this sterilizable containment containing the implantable medical device can be sterilized. Reopening of the containment in order to remove the sterile implantable medical device should only occur immediately prior to implantation.

Each of the contacts of the inner packaging is configured and arranged to provide electrical conductivity or capacitive coupling from outside of the inner packaging to the inside of the inner packaging.

The invention is characterized in, that the inner packaging contains electrically conducting media that provides electrical conductivity or capacitive coupling between the contacts of the inner packaging and an implantable medical device contained inside in a packaging when the containment is filled.

The electric feedthroughs of the outer packaging and the electric contacts of the inner packaging allow to deliver a electrical charge to the medium inside the inner packaging and to measure the impedance or a electric field between the contacts of the inner packaging, which depends on the medium contained in the inner packaging that is influenced by the implantable medical device. Thus, it is possible to communicate galvanically with an implantable medical device even when the implantable medical device is contained in the containment and the inner and the outer packaging are closed. Thus, communication with the implantable medical device can occur without opening the inner and the outer packaging, so that the implantable medical device can be kept sterile while, for instance, programming the implantable medical device.

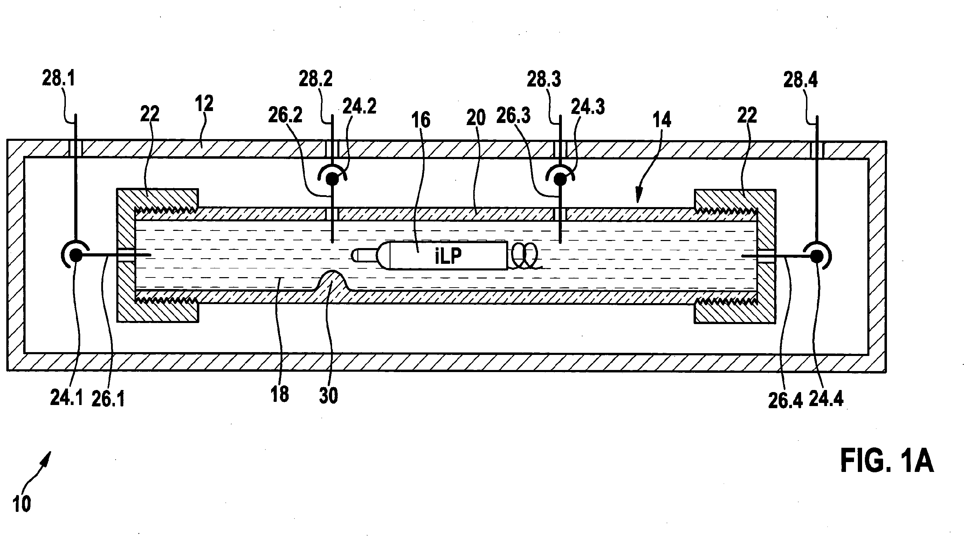

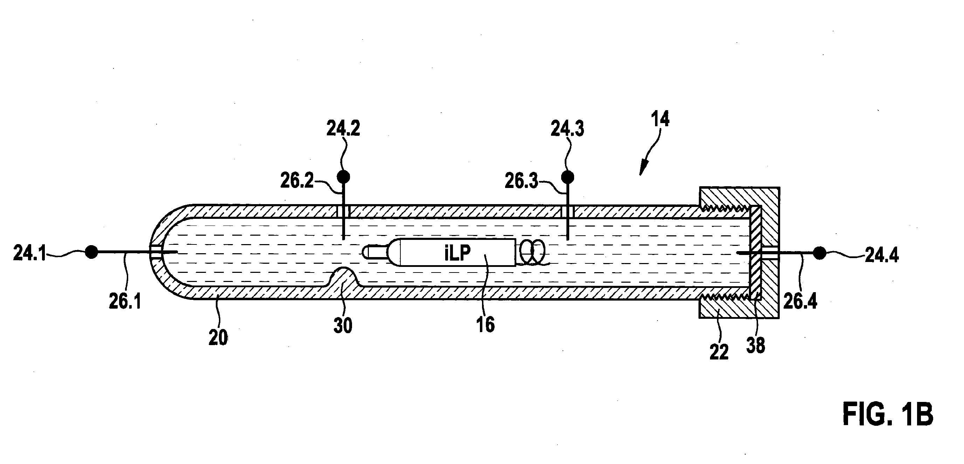

The inventors of the present invention have recognized that there is a need for creative new packaging, module, and/or programmer considerations to enable such flexibilities to enable galvanic programming (and/or activation) of the device within the sterile package. Some ideas involve the use of metal connections (possibly foil or electrode pins) that pass through the packaging and interface with the device. The interfacing with the device can involve direct connections, capacitive coupling, or even impedance-based strategies. One option is to use a cavity filled with a conductive fluid or gel that can house the device and provide an electrical connection to a packaged medical implant; c.f. the embodiment of Fig. 1. A programmer can then connect to the feedthroughs metal tabs on the outer surface of the outer packaging and because of the electrical connection to the device, it is possible to activate and even program the implant galvanically without having to remove it from a sterile containment.

This invention facilitates galvanic communication with an implantable medical device, e.g. an ILP prior to implantation while maintaining the implantable medical device within sterile packaging. Some implantable leadless pacemakers employ an impedance-based means for enabling communication between the implant and the programmer. This strategy, referred to as passive galvanic communication, utilizes a dynamically-controlled switch (located within the implantable medical device) to modify the implantable leadless pacemaker's electrode impedance. This modification of the electrode impedance can be modulated by the implantable medical device and detected by either distally-stationed receiver electrodes (typically ECG pads or skin surface electrodes) or via direct contact with the implantable medical device. This principle can be also used for the opposite communication direction, i.e. a modulated impedance between the electrodes of the external device is detected by the implantable medical device.

Another method of communication, referred to as active galvanic communication, is based on the transmission of voltage or current pulses by electrodes of the transmitting device that are galvanically conducted by the body to electrodes of the receiving device. In both cases, galvanic or electrical conductive paths between the implantable medical device and the external device are required. By enabling such conductive paths the outlined invention thus supports the activation, programming, and interrogation of implantable medical devices after sterilization and packaging procedures have been completed. The invention additionally enables developer support for non-invasive/non-destructive device debug/testing.

Presently, there is no available solution for enabling galvanic communication with an implant in a manner that 1.) avoids breaching of its sterile packaging or 2.) runs acute risk for contaminating the sterility of the implantable medical device prior to installation within the patient receiving therapy. Communication between the implantable medical device and the programmer, when the implantable medical device is stationed outside of the cardiac volume, typically leverages either 1.) placement of the implantable medical device into a conductive water bath with appropriate electrical connections or 2.) direct electro-mechanical interfacing with the implant's electrodes.

### Alternative solutions and drawbacks

Lacking the solution offered by the present invention, data exchange with the implant must be performed 1.) using special equipment prior to device sterilization and packaging procedures; 2.) within a sterile field, again using special equipment that unnecessarily crowds the clinical environment and adds risk of contamination; or 3.) subsequent to routing the implantable medical device through the patient's vasculature and into the cardio vascular volume or in general into the body where it can relay data to skin-mounted conductive pads. In cases where an implantable medical device is either permanently contaminated or damaged as a result of alternative strategies for communication support, it would prove necessary to scrap the implantable medical device and unpack another replacement implantable medical device to provide leadless therapy. Furthermore, contamination cannot be easily detected during an implant procedure, so it may likely lead to an infection. Such waste demands accommodation for yield loss and customer support management that proves highly political and unnecessarily challenging.

Placing the implantable medical device within a dedicated conductive saline bath demands assurances that either 1.) the saline bath is sterile (if the implant is to be implanted following such treatments); or 2.) additional procedures will be run to re-sterilize the implantable medical device prior to its use in delivering patient therapies. To maintain a sterile saline bath means that precious real estate within the clinical sterile field is unfortunately consumed. Conversely, added sterilization steps after opening a sterile implantable medical device own the potential for increasing patient infection risks, extending the duration of implant procedures, risk of damaging the device if improper sterilization parameters are used, and again demanding the placement of special equipment within the sterile field to return the implant to an implantable condition. Such procedures can even nullify the intent of sterile packaging. Re-sterilization is contra-indicated in most active implantable medical device labeling. Employing direct electro-mechanical interfacing with the implantable medical device's electrodes also runs the risk for implantable medical device contamination (via excess handling) and furthermore arrives with an increased potential for damaging the implantable medical device's electrodes. The implantable medical device's electrodes are typically covered with fractal coatings and carefully-defined exposed active areas. Mechanical abrasions associated with deliberate electro-mechanical contact can undesirably alter their capabilities for pacing, sensing, and supporting galvanic communication.

In contrast thereto, inventors propose packaging implantable medical devices in sterile containment that enables galvanic data exchange with a programmer while the containment is sealed.

As can be taken from the foregoing, it is preferred that the implantable medical device is configured to allow a galvanic communication. An implantable medical device that can communicate galvanically comprises means that the implantable medical device can influence a small electric field that is imposed on the medium surrounding the implantable medical device. Passive galvanic communication can be achieved by providing an implantable medical device that comprises a means to alternatingly establish and disconnect a connection between two electrode poles of the implantable medical device to thus alter the impedance between the two electrodes and the impedance of the medium containing the implantable medical device. In other embodiments, the implantable medical device can be configured to generate a small local electric field that is detectable or influences an imposed electric field.

Preferably, the electric conducting medium contained in the inner packaging is a fluid or a liquid gel, for instance saline or a conductive gel. Alternatively, the conductive medium can be a conductive solid material, for instance a conductive foam or potentially even steel wool. In case the conductive medium is a solid material, the solid material is arranged within the inner packaging so as to conduct electric current or to allow propagation of an electric field between the contacts of the inner packaging and the electrode poles of the implantable medical device within the inner packaging.

In a further embodiment, the outer packaging comprises four feedthroughs, and the inner packaging comprises four contacts. This allows using two feedthroughs together with two contacts for imposing an electric field on the medium in the inner packaging. The other two feedthroughs and the corresponding contacts can be used for signal response measurement that is for current or voltage or impedance or electric field measurement, respectively. The resulting 4-point measurement has the advantage over just two feedthroughs of eliminating the signal loss through the packaging from the measured signal.

Preferably, the contacts of the inner packaging are configured as inner feedthroughs that can feed connections from outside of the inner packaging to the electrically conducting medium inside the inner packaging. As such, the contacts can be part of a conductor that terminates inside the inner packaging. Alternatively, the contacts and the feedthrough provided by the contacts of the inner packaging are configured to allow a capacitive coupling between the contact and the electrically conducting medium inside the inner packaging. According to preferred embodiments of the containment, the inner packaging comprises means to hold the implantable medical device in place so it cannot move around inside the inner packaging when the inner packaging is closed. The means to hold the implantable medical device in place can, for example, be protruding contour elements of one or more walls of the inner packaging.

Preferably, the inner packaging comprises means for capacitive coupling to an implantable medical device's communication electrode inside the inner packaging. This eliminates the need for at least one inner feedthrough extending through the wall of the inner packaging.

In the preferred embodiment the inner packaging comprises a cavity which contains the medical device and the conductive medium for galvanic communications. In an alternate embodiment the inner packaging comprises a tube or vial with at least one sealing cap. Thus, the inner packaging can easily be opened without touching the implantable medical device or the conductive medium surrounding the implantable medical device. This helps to keep the implantable medical device sterile.

In a further embodiment the inner packaging comprises at least one protrusion to assist centering an implantable medical device within the inner packaging or to hold an implantable medical device within the inner packaging in place. This helps to achieve consistent conditions for data communication with the implantable medical device.

In a further embodiment either some or all of the contacts or some or all of the outer feedthroughs or both are configured as spring contacts, e.g. pogo pins. Thus, reliable contact between the electrically conducting elements is achieved.

Preferably, the sterilizable containment contains an implantable medical device within the inner packaging.

The implantable medical device preferably is an implantable leadless pacemaker.

The implantable medical device preferably is configured for impedance-based data communication with an external device.

According to a further aspect of the invention, a method of programming and/or testing an implantable medical device is provided. The method comprises the steps of:
- placing the implantable medical device into a containment as disclosed herein,
- closing the containment containing the implantable medical device,
- sterilizing the containment containing the implantable medical device, and
- programming and/or testing the implantable medical device within the closed containment.

This invention provides a compact, ready-made means for communicating with an implantable medical device without mandating that such data exchange necessarily occurs after the implantable medical device is stationed within patient vasculature. It supports debug, test, activation, and programming capabilities without any need to remove the implant from sterile packaging. For peace of mind purposes, this demonstrative capability can aid in adoption of leadless therapies, as clinicians can easily see the device in an operational state without needing to initiate a surgical procedure. From a quality assurance vantage, the manufacturer can additionally perform "up till the last possible minute" device evaluations, prior to sending them through the distribution channels.

### Brief Description of the Drawings

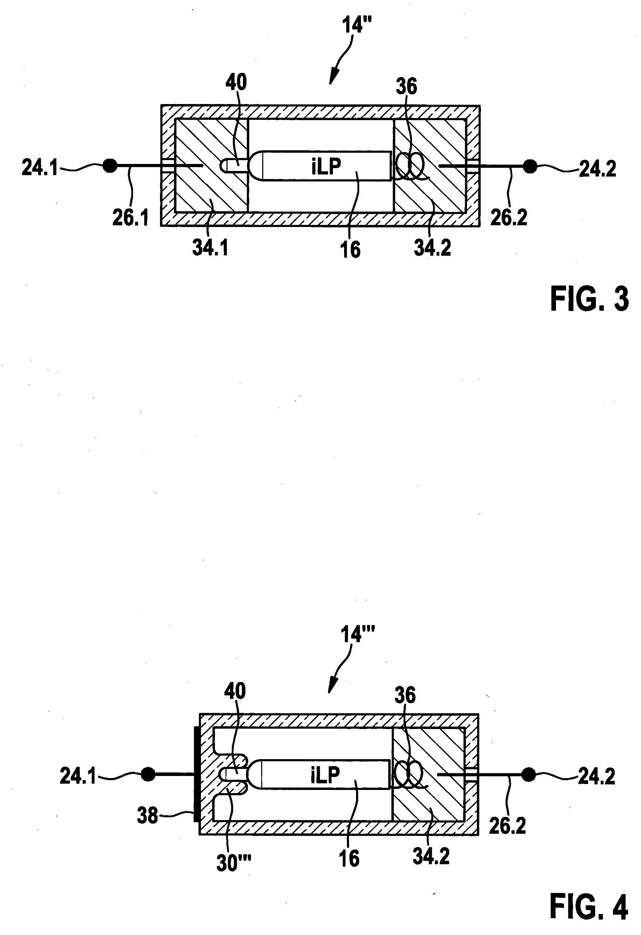

The above and other aspects, features and advantages of the present invention will be more apparent from the following more particular description thereof, presented in conjunction with the following drawings. Four distinct embodiments of the invention are illustrated in detail. In two of them, an inner packaging of the sterile containment surrounds the implantable medical device with a electrically conductive solution and electrical feedthroughs provide contacts to which the programmer can connect for data transmission/receipt. In another embodiment (Fig. 3), controlled electro-mechanical feedthroughs, native to the packaging, offer a data conduit. Last, built-in capacitive coupling hardware, again, native to the packaging, enables dialogue between the implant and the programmer (Fig. 4).

Of the Figures,
- Fig. 1A: illustrates a first embodiment of an inventive containment, containing an implantable medical device;
- Fig. 1B: illustrates an alternative of the a first embodiment of an inventive containment, containing an implantable medical device;
- Fig. 2: illustrates an alternative embodiment of an inventive containment, containing an implantable medical device;
- Fig. 3: illustrates an alternative inner packaging of a containment according to the invention;
- Fig. 4: discloses a further alternative inner packaging of a containment according to the invention;
- Fig. 5: discloses an alternative embodiment of the containment shown in Fig. 2; and
- Fig. 6: discloses the embodiment of the containment shown in Fig. 5, with the outer packaging opened.

### Detailed Description of the Invention

Fig. 1A is a cross-sectional view of the first embodiment of a containment 10 according to the invention. The containment 10 comprises an outer packaging 12 and an inner packaging 14. The inner packaging 14 is enclosed by the outer packaging 12. Within the inner packaging 14, an implantable medical device 16 is contained. The implantable medical device 16 is an implantable leadless pacemaker (ILP) that can be programmed by way of galvanic or impedance-based communication.

To allow such galvanic or impedance-based communication, the inner packaging 14 further contains an electrically conductive medium 18, such as saline or a liquid conductive gel, in which the implantable medical device 16 is embedded.

In the embodiment of Fig. 1A, the inner packaging 14 comprises a tube 20 and two sealing caps 22 on both longitudinal ends of tube 20. Tube 20 may be made from glass, although it could also be made from polymers.

In an alternative embodiment, the inner packaging 14 shown in Fig. 1B. comprises a tubular body 20, wherein on of the longitudinal end is closed and the other longitudinal end is closed with a sealing cap 22. In both embodiments the sealing caps 22 may be screw-on caps and may further comprise a gasket (38) to enable viable sealing.

Inner packing 14 features at least two, in the embodiments of Fig. 1A and Fig. 1B four electric contacts 24.1, 24.2, 24.3 and 24.4. Each contact 24.1, 24.2, 24.3 and 24.4 is part of a respective inner feedthrough 26.1, 26.2, 26.3 and 26.4, which penetrates tube 20 or cap 22, respectively, so that inner feedthroughs 26.1, 26.2, 26.3 and 26.4 each have electric contact to the medium 18 within inner packaging 14.

Outer packaging 12 has four outer feedthroughs 28.1, 28.2, 28.3 and 28.4, each of which corresponds with one of the inner contacts 24.1, 24.2, 24.3 and 24.4. When both the inner packaging 14 and the outer packaging 12 are closed, outer feedthroughs 28.1, 28.2, 28.3 and 28.4 each have direct electric contact with a corresponding inner contact 24.1, 24.2, 24.3 and 24.4. To provide reliable electric contact in the closed state of the containment, either one of the two parts that contact each other may be a pogo pin or a similar spring contact. In another embodiment these contacts are made by pressing two conductive foil windows (one of the outer and inner package) together with a pattern of micro ridges. The foil sheets can easily be separated when the outer package is opened.

In the embodiment depicted in Fig. 1A, two of the outer feedthroughs and two of the inner contacts, namely feedthroughs 28.1 and 28.4 together with contacts 24.1 and 24.4 are used to inject communication current or to impose a small electric field, while the other outer feedthroughs 28.2 and 28.3 together with the corresponding contacts 24.2 and 24.3 are used as electrodes to measure a response from the implantable medical device 16. Thus, when both the inner packaging 14 and the outer packaging 12 are closed and enclose the implantable medical device 16, a galvanic communication is possible, further, even when the outer packaging is removed and only the inner packaging is still sealed, a communication is possible.

On the inside of tube 20, a protrusion or bump 30 is provided that shall have to position the implantable medical device 16 right in the center of tube 20. In case the implantable medical device 16 has moved within the inner packaging 14, containment 10 can be tilted to reposition the implantable medical device 16 so it abuts bump 30.

It is to be appreciated that the containment may feature only two outer feedthroughs and two contacts of the inner packaging. In such embodiment, injecting a current or imposing a small electric field and measuring the response from the implantable medical device 16 would occur over the same two inner and outer feedthroughs.

The embodiment of Fig. 1A involves the placement of the implantable medical device 16 (e.g. implantable leadless pacemaker) within a saline or conductive gel filled vial or tube 20. The tube 20 presents bottle threads at one end or both ends. To cap the tube end(s), one or two screw-on lids or sealing caps 22 each with an electrical feedthrough 26.1 and 26.4 provide means for injecting the comunication current or to impose a small electic field. Two additional feedthroughs 26.2 and 26.3 extend through either the end or the side of the tube 20 to provide connections 24.2 and 24.3 for measuring response signals of the implantable medical device 16. In the embodiments of Fig. 1A and Fig. 1B, the inner packaging 14 (in particular tube 20) employs at least one "bump" feature 30 that allows the physician to tilt the tube 20 to one end and have the implantable medical device center itself between the feedthroughs 26.2 and 26.3. Alternatively multiple bumps would be employed to keep the medical device in the optimal location without tilting. This alignment optimizes the measurement of the response from the implantable medical device 16 and because the "bump" 30 is only on one side of the implantable medical device 16 simply unscrewing one cap 22 of the inner packaging allows the implantable medical device 16 to be removed from the inner packaging 14 without any need to break or cut into the saline-containing tube 20 (i.e. avoids any need for special tools). There is also potential flexibility for removing the outer-most feedthroughs to further simplify the packaging design. Surrounding the inner packaging 14 is an outer packaging 12 that additionally provides electrical feedthroughs 28.1, 28.2, 28.3 and 28.4 capable of supporting communication. Using pogo-pin or similar design features it proves possible to enable electrical connections from outside the outer packaging 12 all the way into the core of the inner packaging 14 where the implantable medical device 16 is located.

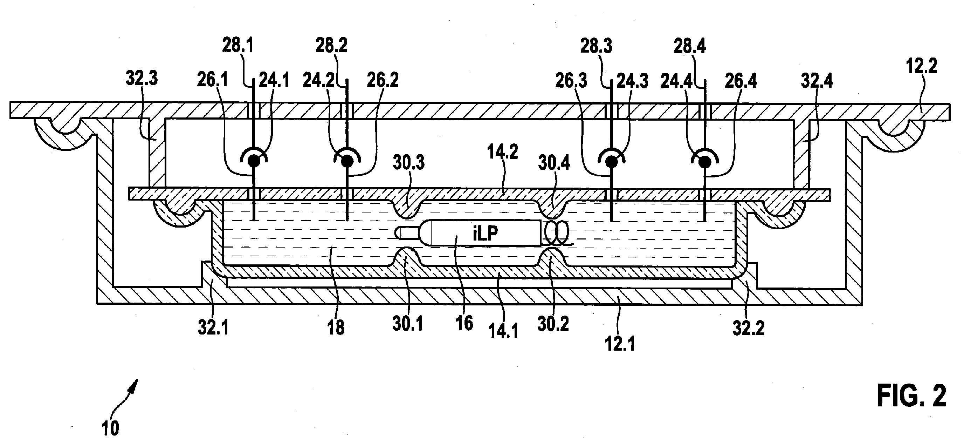

The embodiment of Fig. 2 differs from the embodiment of Fig. 1 in that the inner packaging 14' and the outer packaging 12' are both designed as blister packages, each having a bottom part 12.1 and 14.1, respectively, and a lid part 12.2 and 14.2, respectively. The lid parts 12.2 and 14.2 can be peeled off manually in order to open the outer packaging and the inner packaging, respectively.

As can be taken from Fig. 2, outer feedthroughs 28.1, 28.2, 28.3 and 28.4 are arranged to extend through lid part 12.2 of outer packaging 12. Likewise, contacts 24.1, 24.2, 24.3 and 24.4 are arranged on lid part 14.2 of inner packaging 14'. Again, inner packaging 14' is filled with a liquid or semi-liquid electrically conducting medium, such as saline or electrically conducting gel. Similar to the embodiment of Fig. 1, electrical feedthroughs 26.1, 26.2, 26.3 and 26.4 pass through the inner packaging to enable data exchange. Those same feedthroughs 26.1, 26.2, 26.3 and 26.4 link to an additional set of outer feedthroughs 28.1, 28.2, 28.3 and 28.4 on an outer blister packaging 12'. Communication is thus supported through all layers of the double sterile containment.

Bottom part 14.1 and lid part 14.2 of the inner packaging 14' both feature protrusions 30.1, 30.2, 30.3 and 30.4 extending into the interior of inner packaging 14' in order to hold the implantable medical device 16 in place. Similarly, bottom part 12.1 and lid part 12.2 of outer packaging 12' both comprise inwardly extending protrusions 32.1, 32.2, 32.3 and 32.4 in order to hold inner packaging 14' in place with outer packaging 12'. Thus, it is ensured that outer feedthroughs 28.1, 28.2, 28.3 and 28.4 will reliably contact contacts 24.1, 24.2, 24.3 and 24.4 of inner packaging 14'.

Again, communication with the implantable medical device 16 is possible while keeping outer packaging 12 and inner packaging 14 closed, so that implantable medical device 16 is kept sterile.

Similar to the embodiment of Fig. 1A and Fig. 1B, communication with the implantable medical device 16 is also possible when outer packaging 12' is opened but inner packaging 14' is still closed.

Fig. 3 shows a third embodiment of an inner packaging 14". Inner packaging 14" can be placed within an outer packaging similar to the embodiments of Figs. 1 and 2.

In contrast to the embodiments of Figs. 1 and 2, inner packaging 14" of Fig. 3 is not filled with a liquid electrically conducting medium. Instead, two parts 34.1 and 34.2 made from conductive solid material are provided inside inner packaging 14", the conductive solid material is configured to contact communication electrodes of the implantable medical device 16 and to further hold implant medical device 16 in place. Contacts 24.1" and 24.2" of inner packaging 14" together with inner feedthroughs 26.1" and 26.2" are arranged and configured to establish an electric connection between contacts 24.1" and 24.2" and the two pieces 34.1 and 34.2 of conductive solid material inside inner packaging 14".

Thus, the embodiment of Fig. 3 includes an electromechanical means for enabling communication. Within the inner packaging 14", an anchor 36 acting as first communication electrode of the implantable medical device 16 is inserted into the conductive solid material (likely realized as a foam, a hydrogel, or something akin to steel wool). A separate piece of this same (or similar) conductive solid material is also used to surround the second communication electrode 40 of the implantable medical device 16. Electrical feedthroughs 26.1" and 26.2" associated with the inner packaging 14" then connect to these two independent parts 32.1 and 32.2 of conductive material to provide galvanic connection.

Fig. 4 shows still another alternative embodiment on an inner packaging 14'" that can be placed within an outer packaging, as disclosed in Figs. 1 and 2. Inner packaging 14"' of Fig. 4 features one piece 34.2 of electrically conductive solid material inside inner packaging 14"' similar to the embodiment of Fig. 3. Thus, direct electric connection between contact 24.2 and one of the communication electrodes of implantable medical device 16 is established.

For contacting the other communication electrode 40 of implantable medical device 16, no feedthrough is provided as in the previous embodiments, but rather an electrode 38 (coupling plate) connected to contact 24.1 is provided for capacitively coupling to the corresponding communication electrode of the implantable medical device 16.

Again, inner packaging 14"' is featuring an inwardly extending protrusion 30'" to hold the implantable medical device 16 in place.

As can be seen, the embodiment of Fig. 4 reduces the need for inner feedthroughs to a single feedthrough 26.1. This leads to a package design simplification, which provides both, easy manufacturing and reliable sealing of the packaging of the containment.

As a simplified further embodiment of the first two embodiments, the electrical interfacing with the implantable medical device's second communication electrode 40 may be supported via capacitive coupling. This strategy eliminates one of the inner packaging feedthroughs and simplifies the overall packaging design. It may even prove possible to couple to the electrode 40 from outside of the outer packaging, eliminating the need to station a coupling plate 38 on the inner packaging 14"'.

Another simplified offshoot is to use capacitive coupling rather than a feedthrough to pass the signal through the hermetic package. Once inside the package, electrical contacts can then carry the signal to the device electrodes. This is different from Fig. 4, where the capacitive couple is directly to the implant rather than to metal inside the package which contact the electrode.

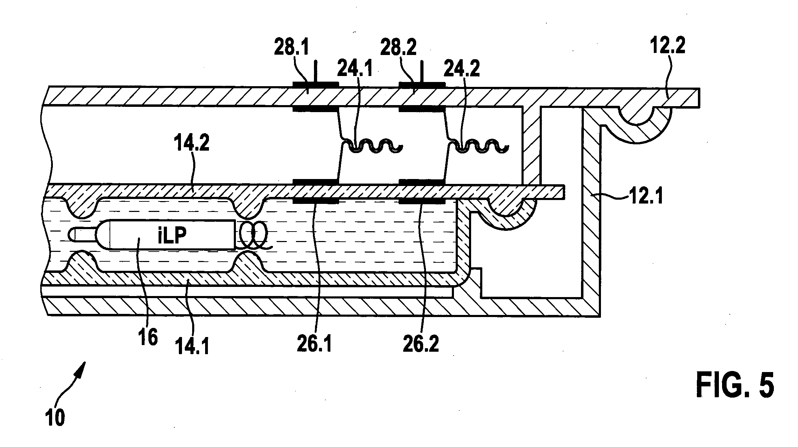

In a further embodiment similar to the embodiment shown in Fig. 2, inner and outer feedthroughs are designed as capacitors. In this embodiment depicted in Fig. 5, the outer packaging 12 and the inner packaging 14 are as in the embodiment of Fig. 2 both designed as blister packages, each having a bottom part 12.1 and 14.1, respectively, and a lid part 12.2 and 14.2, respectively. The lid parts 12.2 and 14.2 can be peeled off manually in order to open the outer packaging and the inner packaging, respectively. In Fig. 5, only the right part of the containment is shown.

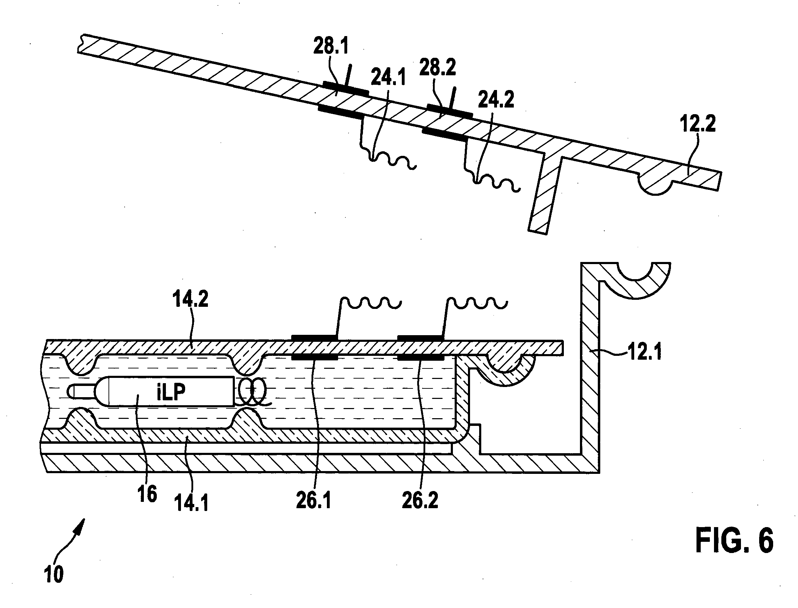

As can be taken from Fig. 5, outer feedthroughs 28.1 and 28.2 and inner feedthroughs 26.1, 26.2 are arranged as capacitive couplings through lid part 12.2 of outer packaging 12 and through lid part 14.2 of inner packaging 14 respectively. To enable capacitive coupling, inner and outer feedthroughs comprise metallic foil pads arranged at the outer and inner side of inner and outer lid part 12.2 and 14.2 respectively. Two opposite metallic foil pads and the dielectric material of lid parts form a capacitor. The capacitors of the outer and inner packaging form the feedthroughs and are connected by contacts 24.1 and 24.2. Contacts 24.1 and 24.2 are made by pressing two conductive foil windows (one of the outer and inner package) together with a pattern of micro ridges. The foil sheets can easily be separated when the outer package is opened as shown in Fig. 6. Again, inner packaging 14' is filled with a liquid or semi-liquid electrically conducting medium, such as saline or electrically conducting gel. Communication is thus supported through all layers of the double sterile containment.

In this embodiment, communication, in particular using small electric fields, with the implantable medical device 16 is possible while keeping outer packaging and inner packaging closed, so that implantable medical device 16 is kept sterile.

Similar to the embodiment of Fig. 1A and Fig. 1B, communication with the implantable medical device 16 is also possible when outer packaging is opened but inner packaging is still closed.

In yet another embodiment, feedthroughs for galvanic communication are included through the inner package, but not the outer package. Rather, long steralizable wires are included inside the outer package which connect to the feedthroughs of the inner package. When a user want to communicate with the device prior to implant, the user would open the outer package, and then run the sterile wire to a device programmer outside of the sterile field. This is similar to how programming wands are used today. The wands are inside of the steral field during implants, but cables run from the wand to a medical device programmer outside of the sterel field. The advantage of this implementation is that less feedthroughs are required.

Regardless of the specific embodiment, after packaging the implantable medical device in double containment, the entire assembly proceeds through a sterilization process. As some of the packaging embodiments (glass tube, and full devices emersion in saline or gel) present unique challenges to ETO and/or Sterad sterilization processes, it may prove optimal to employ either gamma irradiation and/or ultra-violet exposure techniques - both of which are common to medical products in commercial use.

### Reference list

- 10: containment
- 12: packaging
- 14: inner packaging
- 16: medical device
- 18: conductive medium
- 20: tube
- 22: caps
- 24.1, 24.2, 24.3, 24.4.: electric contact
- 26.1, 26.2, 26.3, 26.4.: inner feedthrough
- 28.1, 28.2, 28.3, 28.4: outer feedthrough
- 30: bump
- 30.1, 30.2, 30.3, 30.4: protrusion
- 32.1, 32.2, 32.3, 32.4: extending protrusion
- 34.1, 34.2: parts made from conductive solid material
- 36: anchor
- 38: gasket
- 40: electrode

## Claims

1. Sterilizable containment (10) comprising: an inner packaging (14) and an outer packaging (12) that encloses said inner packaging (14), said outer packaging (12) comprising at least two electric feedthroughs (28.1, 28.2) and said inner packaging comprising at least two electric contacts (24.1, 24.2), each of the contacts (24.1, 24.2) of the inner packaging (14) matching one of the feedthroughs (28.1, 28.2) of the outer packaging (12) to provide an electric connection between a respective feedthrough and the corresponding contact when the inner packaging (14) and the outer packaging (12) are closed, each of the contacts (24.1, 24.2) being further configured and arranged to feed electrical charge and/or voltage drops from outside the inner packaging (14) to the inside of the inner package (14) and vice versa,
**characterized in, that**
said inner packaging (14) further containing an electrically conducting medium (18) that allows propagation of electrical charge and/or application of a voltage delta between said contacts (24.1, 24.2) of the inner packaging (14) and an implantable medical device (16) contained in said inner packaging (14) when said containment (10) is filled with such implantable medical device.

2. Sterilizable containment according to claim 1, wherein the electrically conducting medium (18) is a fluid.

3. Sterilizable containment according to claim 1 or 2, wherein the outer packaging (12) comprises four feedthroughs and the inner packaging comprises four contacts.

4. Sterilizable containment according to at least one of claims 1 to 3, wherein the contacts (24.1, 24.2, 24.3, 24.4) of the inner packaging (14) are part of inner feedthroughs (26.1, 26.2, 26.3, 26.4) that can feed electrical charge and/or voltage differences from the outside of said inner packaging (14) to the electrically conducting medium (18) inside the inner packaging (14).

5. Sterilizable containment according to at least one of claims 1 to 4, wherein said electric feedthroughs are capacitors.

6. Sterilizable containment according to at least one of claims 1 to 4, wherein the inner packaging comprises means (38) for capacitive coupling to an implantable medical device's communication electrode inside the inner packaging.

7. Sterilizable containment according to at least one of claims 1 to 6, wherein the inner packaging comprises a tube (20) or vial with at least one sealing cap (22).

8. Sterilizable containment according to at least one of claims 1 to 7, wherein the inner packaging comprises at least one protrusion (30) to assist centering an implantable medical device within the inner packaging or to hold an implantable medical device within the inner packaging in place.

9. Sterilizable containment according to at least one of claims 1 to 8, wherein either some or all of the contacts (24.1, 24.2, 24.3, 24.4) or some or all of the outer feedthroughs (28.1, 28.2, 28.3, 28.4) or both are configured as spring contacts.

10. Sterilizable containment according to at least one of claims 1 to 9, containing an implantable medical device (16) within the inner packaging (14).

11. Sterilizable containment according to claim 10, wherein the implantable medical device (16) is an implantable leadless pacemaker.

12. Sterilizable containment according to at least one of claims 10 or 11, wherein the implantable medical device (16) is configured for galvanic or electric field based data communication with an external device.

13. Sterilizable containment according to at least one of claims 10 or 11, wherein the implantable medical device (16) is configured to be stored in shelf mode and to be activated to an active mode prior to implantation.

14. A method of communicating with an implantable medical device, said method comprising:
- placing the implantable medical device into a containment according to at least one of claims 1 to 13,
- closing the containment containing the implantable medical device
- sterilizing the containment containing the implantable medical device
- programming and/or testing the implantable medical device within the closed containment.

## Patentansprüche

1. Sterilisierbarer Behälter (10), umfassend: eine innere Verpackung (14) und eine äußere, die innere Verpackung (14) umschließende Verpackung (12), wobei die äußere Verpackung (12) mindestens zwei elektrische Durchführungen (28.1, 28.2) umfasst, und die innere Verpackung mindestens zwei elektrische Kontakte (24.1, 24.2) umfasst, wobei jeder der Kontakte (24.1, 24.2) der inneren Verpackung (14) einer der Durchführungen (28.1, 28.2) der äußeren Verpackung (12) zugeordnet ist, um eine elektrische Verbindung zwischen einer betreffenden Durchführung und dem zugeordneten Kontakt zu schaffen, wenn die innere Verpackung (14) und die äußere Verpackung (12) geschlossen sind, wobei jeder der Kontakte (24.1, 24.2) ferner so ausgelegt und angeordnet ist, dass er eine elektrische Ladung und/oder Spannungsabfälle von außerhalb der inneren Verpackung (14) in das Innere der inneren Verpackung (14) und umgekehrt speist,
**dadurch gekennzeichnet, dass**
die innere Verpackung (14) ferner ein elektrisch leitendes Medium (18) enthält, das eine Übertragung der elektrischen Ladung und/oder die Anlegung eines SpannungsDifferenz zwischen den Kontakten (24.1, 24.2) der inneren Verpackung (14) und einer in der inneren Verpackung (14) enthaltenen implantierbaren medizinischen Vorrichtung (16) ermöglicht, wenn der Behälter (10) mit der implantierbaren medizinischen Vorrichtung gefüllt ist.

2. Sterilisierbarer Behälter nach Anspruch 1, wobei das elektrisch leitende Medium (18) ein Fluid ist.

3. Sterilisierbarer Behälter nach Anspruch 1 oder 2, wobei die äußere Verpackung (12) vier Durchführungen umfasst und die innere Verpackung vier Kontakte umfasst.

4. Sterilisierbarer Behälter nach mindestens einem der Ansprüche 1 bis 3, wobei die Kontakte (24.1, 24.2, 24.3, 24.4) der inneren Verpackung (14) Teil der inneren Durchführungen (26.1, 26.2, 26.3, 26.4) sind, die eine elektrische Ladung und/oder Spannungsunterschiede von außerhalb der inneren Verpackung (14) in das elektrisch leitende Medium (18) innerhalb der inneren Verpackung (14) einspeisen können.

5. Sterilisierbarer Behälter nach mindestens einem der Ansprüche 1 bis 4, wobei die elektrischen Durchführungen Kondensatoren sind.

6. Sterilisierbarer Behälter nach mindestens einem der Ansprüche 1 bis 4, wobei die innere Verpackung eine Vorrichtung (38) zur kapazitiven Kopplung mit einer Kommunikationselektrode der implantierbaren medizinischen Vorrichtung innerhalb der inneren Verpackung umfasst.

7. Sterilisierbarer Behälter nach mindestens einem der Ansprüche 1 bis 6, wobei die innere Verpackung ein Röhrchen (20) oder eine Ampulle mit mindestens einer Dichtungskappe (22) umfasst.

8. Sterilisierbarer Behälter nach mindestens einem der Ansprüche 1 bis 7, wobei die innere Verpackung mindestens einen Vorsprung (30) umfasst, um die mittige Anordnung einer implantierbaren medizinischen Vorrichtung innerhalb der inneren Verpackung zu erleichtern oder um eine implantierbare medizinische Vorrichtung innerhalb der inneren Verpackung an Ort und Stelle zu halten.

9. Sterilisierbarer Behälter nach Anspruch 1 bis 8, wobei einige oder alle der Kontakte (24.1, 24.2, 24.3, 24.4) und/oder einige oder alle der äußeren Durchführungen (28.1, 28.2, 28.3, 28.4) oder beide als Federkontakte ausgestaltet sind.

10. Sterilisierbarer Behälter nach mindestens einem der Ansprüche 1 bis 9, der eine implantierbare medizinische Vorrichtung (16) innerhalb der inneren Verpackung (14) enthält.

11. Sterilisierbarer Behälter nach Anspruch 10, wobei die implantierbare medizinische Vorrichtung (16) ein implantierbarer sondenloser Schrittmacher ist.

12. Sterilisierbarer Behälter nach mindestens einem der Ansprüche 10 oder 11, wobei die implantierbare medizinische Vorrichtung (16) für eine auf einem galvanischen oder elektrischen Feld basierende Datenübertragung mit einer externen Vorrichtung ausgelegt ist.

13. Sterilisierbarer Behälter nach mindestens einem der Ansprüche 10 oder 11, wobei die implantierbare medizinische Vorrichtung (16) dafür ausgelegt ist, im Lagermodus aufbewahrt zu werden und vor der Implantierung in einen aktiven Modus aktiviert zu werden.

14. Verfahren zum Kommunizieren mit einer implantierbaren medizinischen Vorrichtung, wobei das Verfahren umfasst:
- Platzieren der implantierbaren medizinischen Vorrichtung in einem Behälter nach mindestens einem der Ansprüche 1 bis 13,
- Schließen des Behälters, der die implantierbare medizinische Vorrichtung enthält,
- Sterilisieren des Behälters, der die implantierbare medizinische Vorrichtung enthält,
- Programmieren und/oder Testen der implantierbaren medizinischen Vorrichtung innerhalb des geschlossenen Behälters.

## Revendications

1. Confinement stérilisable (10) comprenant : un emballage interne (14) et un emballage externe (12), qui inclut ledit emballage interne (14), ledit emballage externe (12) comprenant au moins deux traversées électriques (28.1 , 28.2) et ledit emballage interne comprenant au moins deux contacts électriques (24.1, 24.2), chacun des contacts (24.1, 24.2) de l'emballage interne (14) étant associé à l'une des traversées électriques (28.1 , 28.2) de l'emballage externe (12) pour établir une liaison électrique entre une traversée électrique respective et le contact correspondant lorsque l'emballage interne (14) et l'emballage externe (12) sont fermés, chacun des contacts (24.1, 24.2) étant en outre configuré et agencé pour alimenter en courant électrique et/ou en chutes de tension l'intérieur de l'emballage interne (14) depuis l'extérieur de l'emballage interne (14) et vice versa,
**caractérisé en ce que**
ledit l'emballage interne (14) contient en outre un milieu électriquement conducteur (18) qui autorise une propagation du courant électrique et/ou l'application d'un différences de tension entre lesdits contacts (24.1,24.2) de l'emballage interne (14) et un dispositif médical implantable (16) contenu dans ledit emballage interne (14) lorsque ledit confinement (10) est occupé par un tel dispositif médical implantable.

2. Confinement stérilisable selon la revendication 1, dans lequel le milieu électriquement conducteur (18) est un fluide.

3. Confinement stérilisable selon les revendications 1 ou 2, dans lequel l'emballage externe (12) comprend quatre traversées électriques et l'emballage interne comprend quatre contacts.

4. Confinement stérilisable selon au moins l'une des revendications 1 à 3, dans lequel les contacts (24.1, 24.2, 24.3, 24.4) de l'emballage interne (14) font partie des traversées électriques internes (26.1, 26.2, 26.3, 26.4) qui peuvent alimenter en courant électrique et/ou en différences de tension le milieu électriquement conducteur (18) à l'intérieur de l'emballage interne (14) depuis l'extérieur dudit emballage interne (14).

5. Confinement stérilisable selon au moins l'une des revendications 1 à 4, dans lequel lesdites traversées électriques sont des condensateurs.

6. Confinement stérilisable selon au moins l'une des revendications 1 à 4, dans lequel l'emballage interne comprend des moyens (38) de couplage capacitif vers une électrode de communication d'un dispositif médical implantable à l'intérieur de l'emballage interne.

7. Confinement stérilisable selon au moins l'une des revendications 1 à 6, dans lequel l'emballage interne comprend un tube (20) ou un flacon avec au moins un capuchon scellé (22).

8. Confinement stérilisable selon au moins l'une des revendications 1 à 7, dans lequel l'emballage interne comprend au moins une protrusion (30) pour faciliter le centrage du dispositif médical implantable dans l'emballage interne ou pour maintenir le dispositif médical implantable en place dans l'emballage interne.

9. Confinement stérilisable selon au moins l'une des revendications 1 à 8, dans lequel soit plusieurs, soit l'ensemble des contacts (24.1, 24.2, 24.3, 24.4), soit plusieurs, soit l'ensemble des traversées externes (28.1, 28.2, 28.3, 28.4) ou les deux sont configurés comme des contacts à ressort.

10. Confinement stérilisable selon au moins l'une des revendications 1 à 9, contenant un dispositif médical implantable (16) à l'intérieur de l'emballage interne (14).

11. Confinement stérilisable selon la revendication 10, dans lequel le dispositif médical implantable (16) est un stimulateur cardiaque sans fil.

12. Confinement stérilisable selon au moins l'une des revendications 10 ou 11, dans lequel le dispositif médical implantable (16) est configuré pour une communication des données avec un dispositif extérieur sur la base d'un champ électrique ou galvanique.

13. Confinement stérilisable selon au moins l'une des revendications 10 ou 11, dans lequel le dispositif médical implantable (16) est configuré pour être conservé en mode conservatoire et pour être activé en mode actif avant l'implantation.

14. Procédé de communication avec un dispositif médical implantable, ledit procédé comprenant:
- la mise en place du dispositif médical implantable dans un confinement selon au moins l'une des revendications 1 à 13,
- la fermeture du confinement contenant le dispositif médical implantable
- la stérilisation du confinement contenant le dispositif médical implantable
- la programmation et/ou le test du dispositif médical implantable dans le confinement fermé.
